# EUROPEAN PATENT APPLICATION

(11) **EP 2 418 484 A2**
(43) Date of publication of application: **15.02.2012**
(21) Application number: 10761859.7
(22) Date of filing: 06.04.2010
(51) Int. Cl.: G01N 33/49, G01N 33/92, C12Q 1/68, G01N 33/15

(54) **PRODUCTION PROCESS OF GENDER-SPECIFIC SERUM AND BIOMARKER USING THE SERUM**

(30) Priority: 06.04.2009 KR 20090029597; 24.03.2010 KR 20100026478; 24.03.2010 KR 20100026479
(71) Applicant: Industry-Academic Cooperation Foundation Yeungnam University, Gyeongbuk 712-749 (KR)
(72) Inventor: CHOI, In Ho, Gyeongsan-si Gyeongsangbuk-do 712-080 (KR); LEE, Dong Mok, Daegu 704-370 (KR); CHANG, Jong Soo, Seoul 137-767 (KR); LEE, Hyun Jeong, Suwon-si Gyeonggi-do 441-350 (KR); LEE, Eun Ju, Dalseong-gun Daegu 711-865 (KR); PRATI, Bajracharya, Gyeongsan-si Gyeongsangbuk-do 712-749 (KR); SHIN, Yu Mi, Gyeongsan-si Gyeongsangbuk-do 712-010 (KR)
(74) Representative: Roos, Peter
(86) International application number: PCT/KR2010/002111
(87) International publication number: WO 2010/117198

(57) **Abstract**

The present invention relates to a production process of a gender-specific serum and a biomarker using the serum. More specifically, the present invention uses a fatty acid that exhibits a specific expression pattern in a gender-specific serum as a biomarker not only for diagnosis of obesity or a disease related to obesity, but also for diagnosis of meat quality since the fatty acid promotes differentiation of muscle derived stem cells into adipose cells. In addition, the present invention can establish a research system studying the effects of steroid hormones on the cell culture by using sera separated from blood that is collected from individual mammal carcasses being disposed, and provide important clues for discovering a gene associated with the synthesis of steroid hormone and for developing treatments for human diseases. Further, the present invention may contribute to increased profits derived from producing high quality sera, a reduced cost with treatment for carcass wastes, and promotion of the eco-industry for reducing environmental hormones.

## Description

### [Technical Field]

The present invention relates to a method of preparing a gender-specific serum and a biomarker using the same, wherein the biomarker is a biomarker for diagnosing obesity or an obesity related disease or a biomarker for evaluating a meat quality of livestock.

### [Background Art]

Last year, the import of bovine spongiform encephalopathy (BSE)-related American cattle and cattle products was comprehensively banned, and thus, the fetal bovine serum (FBS) as a major raw material for culturing animal cells was also not imported. Accordingly, most of Korean life science laboratories which had not secured enough FBS in advance, have to stop their experiments

A serum is a composite product in which various materials are mixed, and is used as an additive to a basic cell culture medium. The serum for cell culture includes growth factors, hormones, components that stimulates cells, etc., and is variously used according to the kind of an animal source. In general, however, FBS is the most frequently used serum. The FBS is isolated from an umbilical cord of a calf during pregnancy, and in particular, is used as a raw material in developing vaccines and protein medical supplies of which technology speeds are being accelerated around the world for recent several years, as well as in culturing animal cells, which is a basic step in biotechnology related experiments.

As a serum, a FBS and a bovine calf serum (BCS) are generally used for cell culture. The BCS is harvested from about 16-month old calf. The BCS is different from the FBS in antibody and hormone contents. A total protein content of FBS is 3.6 mg, and a total protein content of BCS is 6.5 mg that is about two times greater than that of FBS. An antibody content of BCS is also about 100 times greater than that of FBS.

FBS is more often used than BCS, and almost all cell lines grow in FBS. Meanwhile, in culturing cell lines that do not require FBS, BCS can be used instead of FBS. In some cases, cell growth is more active in BCS than FBS, and BCS is more economic than FBS.

Other than the sera described above, a human serum and a horse serum are also available. The human serum is used in culturing several human cell lines. The horse serum is obtained from closed flocks of horses, so the collected serum components are uniform whenever the serum is harvested. However, due to a low concentration of polyamine oxidase, the polyamine may not be digested for promoting cell proliferation in several cells.

Korea has a well-established domestic meat industry with an overall annual growth of 10-15%. The number of Korean cattle slaughtered during 2008 was 769,432, 12.5% increase from 2007. Comparing the sex ration of slaughtered cattle, the number of female cattle was 34.3%, 3.2% increase from 2007, the number of male cattle was increased by 1.2% compared to the year of 2007 (41.1%), and the number of castrated cattle was decreased by 4.4% compared to the year of 2007 (27.8%).

The world market for FBS is worth USD 250 million and the Korea FBS market is worth 7 billion won. In the Korea FBS market, American cattle accounts for 85% and Australian and New Zealand cattle accounts for 15%. A serum from Korean cattle has not been produced or not has not been commercialized on sale, and thus, once the serum import is banned, there are few available countermeasures.

Obesity develops when energy intake is greater than energy consumption for a long period of time and excess energy is stored in fat. In a normal state, appetite is controlled by factors derived from the peripheral system, and examples of the factors are leptin and insulin (Schwartz et al, Nature, 404:661-671, 2000). When the body weight increases, leptin and insulin concentrations in blood increase. Thus, the increased leptin and insulin affect hypothalamus as a feeding center to suppress appetite and promote energy consumption, thereby removing the increased body weight.

This normal feedback system for controlling energy balance is a kind of a protection mechanism for preventing body weight increase. However, the feedback system is not appropriately operated in the case of obesity and thus, obesity becomes serious and accompanying diseases also develop (Kopelman et al., Nature, 404:635-643, 2000).

As a livestock feed, household food waste, chaff, and weeds grown in the fields and harvested in weed production seasons are provided to livestock. Although recently, assorted feeds for livestock are being produced at large scales, a feed that is controlled according to an age and a growth phase in compliance with a systemic growth management program is not provided and thus necessary nutrients are not supplied in the right time. Also, in conventional assorted feeds, components ratios are not scientifically systemized according to an appropriate physiological nutrient intake of Korean cattle.

In particular, typically, an artificial assorted feed includes nutrients, such as crude protein, crude fat, crude fiber, crude ash, calcium, or phosphorous, as a drug affecting metabolism of livestock, vitamins as nutritional supplement, calcium, saccharine, blood substitute, minerals, an organic acid, etc.

In the case of artificial assorted feeds, cereals and vegetables are mixed and processed, and the artificial assorted feed is produced at large scales and is on sale as being packaged. Thus, artificial assorted feeds are easily available. Also, an artificial assorted feed that includes additional nutrients, antibiotics, antibiotic materials, and a preservative is also easily available. However, the artificial assorted feeds are expensive. If an antibiotic content of Korean cattle fed with the artificial assorted feed including antibiotics, antibiotic materials, and a preservative, is evaluated as being higher than an antibiotics reference value during slaughter, the cattle is graded as a low level in the stock rating decision reference, or is not evaluated at all. Thus, the cattle sell for relatively low price.

Meanwhile, myogenic satellite cells (MSCs) are precursor skeletal muscle cells located at the endomysial space of skeletal muscle. MSCs proliferate and fuse with each other to form myotubes, which in turn give rise to new muscle fiber. The myogenesis process begins with stimulations, during which MSCs start to express transcriptional factors such as muscle regulator factors Myf5 and MyoD, and, finally, marker genes such as myogenin and myosin heavy chain are expressed in differentiated myotube forming cells. MSCs possess the multipotential capacity to form adipocyte-like cells (ALCs), osteocytes, and nerve cells.

There have been several studies on the molecular mechanism of MSC transdifferentiation into ALCs. The demand of the marbling in the meat for meat animals has increased the research interest in intramuscular adipocytes. It is known that abnormal lipid droplets within the skeletal muscle of human are crucially important in obesity, type 2 diabetes, and cardiovascular disease (Am J Physiol Endocrinol Metab, 284,: E741-747, 2003; Curr Opin Lipidol 9: 231-236, 1998). It is reported that during transdifferentiation, there is an increase in expression of several transcriptional factors related to adipogenesis, CCAAT/enhancer binding protein-alpha, and peroxisome proliferator-activated receptor gamma (PPARγ). Also, PPARγ activation induces mRNA transcripts of CD36 in macrophage (9). FAT/CD36 is a multifunctional receptor expressed in several cell types, and has been proposed as the fatty acid transporter in adipocytes.

### [Disclosure]

### [Technical Problem]

The present invention provides a method of preparing a gender-specific serum by isolating a serum from a mammal blood that is discarded after slaughter and purifying the serum, according to female, male, and castrated male individuals, wherein the serum is used as an additive for providing a protein engaging in cell growth and function, a hormone, and various nutrients in a in vitro experiment.

The present invention also provides a biomarker for diagnosing obesity or obesity related disease including a fatty acid that is specifically expressed in a gender-specific serum and a method of diagnosing obesity or obesity related disease by using the same.

The present invention also provides a biomarker for evaluating a meat quality of livestock including a fatty acid that is specifically expressed in a gender-specific serum and a method of evaluating a meat quality of livestock by using the same.

### [Technical Solution]

According to an aspect of the present invention, a method is provided for preparing a gender-specific serum, wherein the method includes: preparing blood from a mammal that is discarded after slaughter; first centrifuging the prepared blood; tranquilizing after the centrifuging; quickly freezing a collected supernatant; thawing the resultant product at room temperature, followed by second centrifuging; inactivating the supernatant: and filtering the inactivated supernatant.

According to another aspect of the present invention, a method is provided for diagnosing obesity or obesity related disease including a fatty acid that is specifically expressed in a gender-specific serum. In this regard, the fatty acid may include one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

According to another aspect of the present invention, there is provided a composition for treating or preventing obesity or obesity related disease, the composition comprising an inhibitor that prevents synthesis of a fatty acid that is specifically over-expressed in the gender-specific serum. In this case, the fatty acid may include one or more selected from the group consisting of fatty acids selected from arachidic acid (C20:0) and eicosadienoic acid (C20:2n); derivatives of these fatty acids, and salts of these fatty acids.

According to another aspect of the present invention, there is provided a method of screening an agent for treating obesity or obesity related disease, wherein the method includes treating a biological sample obtained from an individual that is needed to prevent or treat the obesity or obesity-related disease with a compound; and detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

According to another aspect of the present invention, there is provided a method of diagnosing obesity or obesity-related disease, wherein the method includes detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids in a biological sample obtained from an individual that is needed to prevent or treat the obesity or obesity-related disease; and determining whether obesity or obesity related disease has been developed by comparing the expression profile with an expression profile of that of a normal control group.

According to another aspect of the present invention, there is provided a biomarker for evaluating a meat quality of livestock, wherein the biomarker includes a fatty acid that is specifically expressed in the gender-specific serum. The fatty acid may include one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

According to another aspect of the present invention, there is provided a method of evaluating a meat quality of livestock, wherein the method includes detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24 :0), and nervonic acid; derivatives of these fatty acids, and salts of these fatty acids in a serum of livestock.

According to another aspect of the present invention, there is provided a feed composition for fattening livestock, wherein the feed composition includes a fatty acid that is specifically over-expressed in the gender-specific serum as an active ingredient. The fatty acid may include as an active ingredient one or more selected from the group consisting of fatty acids selected from arachidic acid (C20:0) and eicosadienoic acid (C20:2n); derivatives of these fatty acids, and salts of these fatty acids.

### [Advantageous Effects]

According to the present invention, a serum is isolated from collected mammal blood that is discarded after slaughter according to different individuals. The sera obtained as above may contribute to establishment of a research system for studying the effect of a steroid hormone on cell culture, may provide a critical clue in discovering a gene that is engaged in hormone synthesis and developing a human disease therapeutic agent. Also, higher revenues due to production of high-quality serum, lower environmental costs caused by consumption of slaughter waste, and activation of the environmental friendly industry that may contribute to a decrease in environmental hormone may also be achievable.

Also, because a fatty acid specifically contained in a gender-specific serum according to the present invention promotes differentiation from muscle stem cells into adipocyte, the fatty acid may be used as a biomarker in diagnosing obesity or obesity-related disease. Furthermore, obesity or obesity-related disease may also be treated or prevented by using an inhibitor that suppresses synthesis of the particular fatty acid promoting differentiation from muscle stem cells into adipocyte,.

Also, because a fatty acid specifically contained in a gender-specific serum according to the present invention promotes induction of differentiation from muscle stem cells into adipocytes or adipocyte like cells, the fatty acid may contribute to marbling production, higher meat quality, and thus high-quality livestock production.

### [Description of Drawings]

FIG. 1 is a flowchart illustrating processes for isolating and purifying a serum according to an embodiment of the present invention.
FIG. 2 shows the effect of different sera prepared according to the present invention on myogenic satellite cell primary culturing.
FIGS. 3 and 4 show the effect of different sera of Korean bovine and porcine individuals on various cell lines proliferation, respectively.
FIG. 5 shows a relationship between lipid accumulation induced from muscle differentiation and a hormone.
FIGS. 6 and 7 respectively show Oil-Red-O staining pictures and an optical density graph of the staining, showing the effect of different sera prepared according to the present invention on differentiation from myogenic satellite cells into adipocyt.
FIG. 8 shows formation of myotube when single cells are fused into a multi-cell in a bovine MSC culture.
FIG. 9 shows Giemsa staining and ORO staining results from which the formation of liquid droplets in the myotube formed in the bovine MSC culture is confirmed.
FIG. 10 shows MSC proliferation effects in media supplemented with a gender-specific serum according to the present invention.
FIGS. 11 and 12 respectively show immunocytochemical analysis results and real-time RT-PCR results showing the effect of the gender-specific serum according to the present invention on myogenesis-related gene expression.
FIG. 13 shows real-time RT-PCR results obtained using a combined E₂ and testosterone treatment, showing the effect of the gender-specific serum according to the present invention on the myogenesis-related gene expression.
FIGS. 14 to 16 show the effect of a gender-specific serum according to the present invention on transdifferentiation from MSC to adipocyte (ALC).
FIGS. 17 to 20 show the effect of a gender-specific serum according to the present invention on transdifferentiation from MSC to adipocyte (ALC), evaluated using a combined E₂ and testosterone treatment.

### [Best Mode]

The present invention provides a method of preparing a gender-specific serum, wherein the method includes: preparing blood from a mammal that is discarded after slaughter; first centrifuging the prepared blood; tranquilizing after the centrifuging; quickly freezing collected supernatant; thawing the resultant product at room temperature, followed by second centrifugation; inactivating the supernatant: and filtering the inactivated supernatant. The gender-specific serum according to the present invention may be any one selected from the group consisting of female serum, male serum, and castrated male serum.

Also, the first centrifugation may be performed at 4,000 to 6,000 rpm for 10 to 30 minutes, and for example, at 5,000 rpm for 20 minutes. The first centrifugation may result in higher cohesiveness. If the centrifugation is carried out under conditions that do not comply with those described above, hemoglobin coagulum may occur.

Also, the tranquilizing may be performed at a temperature of 0 to 10°C for 25 to 30 hours, and for example, at a temperature of 4°C for 25 to 30 hours. If the tranquilizing is carried out under conditions that do not comply with those described above, blood coagulation may be disrupted.

Also, the second centrifugation may be performed at 6,000 to 8,000 rpm for 20 to 40 minutes, and for example, at 7,000 rpm for 30 minutes. The second centrifugation may allow the filtering to be easily performed. If the centrifuging is not performed in this stage, filtering may be difficult.

Also, the inactivation may be performed at a temperature of 45 to 65°C for 20 to 40 minutes, and for example, at a temperature of 56°C for 30 minutes. If the inactivation is carried out under conditions that do not comply with those described above, protein annealing according to temperature change may occur.

Also, the filtering may be performed using a sterilized filter paper, a sterilized syringe filter, or a combination thereof. If the filtering is carried out under conditions that do not comply with those described above, contamination may occur during culture.

A gender-specific serum prepared by using a method according to the present invention may overcome a problem of a steroid hormone study using a conventional fetal bovine serum (FBS). That is, although FBS contains steroid hormone, the steroid hormone content cannot be specified. Accordingly, when other steroid hormones are added, the addition effect was not able to be confirmed. However, in the case of the serum according to the present invention, the steroid hormone content is accurately measurable. Therefore, the effect of the steroid hormone including other steroid hormone may be effectively evaluated.

Also, according to the present invention, the difficulty in filtering for isolating and purifying a serum is overcome by using a filter paper or a syringe filter after quick-freezing and centrifugation, thereby reducing costs. Also, the environmental pollution is preventable due to the reuse of discarded blood of slaughtered mammal.

Also, the present invention also provides a biomarker for diagnosing obesity or obesity related disease including a fatty acid that is specifically expressed in a gender-specific serum.

The fatty acid may include one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

The obesity-related disease may be selected from the group consisting of diabetes, insulin resistant syndrome, hyperlipemia, ostarthritis, lipodystrophy, nonalcoholic steatohepatitis, cardiovascular disease, a polycystic ovary syndrome, and a metabolic syndrome.

The biomarker may promote expression of a FAT/CD36 gene, and is specifically expressed in a gender-specific serum selected from a female serum and a male serum, and for example, in a female serum.

In particular, among gender-specific sera prepared according to an embodiment of the present invention, female serum (FS) promotes differentiation from muscle stem cells into adipocyte, enhances expression of FAT/CD36, and contains a particularly great amount of one or more fatty acid selected from arachidic acid (C20:0) and eicosadienoic acid (C20:2n). Also, the present invention also provides a composition for treating or preventing obesity or obesity related disease including an inhibitor that prevents synthesis of a fatty acid that is specifically over-expressed in the gender-specific serum. The fatty acid may be any one selected from the group consisting of fatty acids selected from arachidic acid (C20:0) and eicosadienoic acid (C20:2n); derivatives of these fatty acids, and salts of these fatty acids. The fatty acid synthesis inhibitor may include a FAT/CD36 inhibitor, which may be selected from the group consisting of siRNA that inhibits expression of a FAT/CD36 gene and an antibody that specifically bonds to the FAT/CD36 gene. Also, the FAT/CD36 inhibitor may suppress induction of differentiation from muscle stem cells into adipocytes or adipocyte like cells.

The siRNA may be prepared by using any known method of preparing a RNA molecule in the art, and the RNA molecule preparation method may be a chemical synthesis method or an enzymatic method. An example of the chemical synthesis method for preparing a RNA molecule is disclosed in the following reference (Verma and Eckstein, Annu. Rev. Biochem. 67, 99-134, 1999), and an example of the enzymatic method of preparing a RNA molecule is a method using a phage RNA polymerase, such as T7, T3, or SP6 RNA polymerase (Milligan and Uhlenbeck, Methods Enzymol. 180: 51-62, 1989).

The composition according to the present invention may further include a carrier, an excipient, or a diluent which are commonly used in preparing a pharmaceutical composition. Examples of a carrier, an excipient, and a diluent that are available for use in the composition according to the present invention are lactose, dextrose, sucrose, sorbitol, mannitol, xylitol, erythritol, maltitol, starch, acacia rubber, alginat, gelatin, calcium phosphate, calcium silicate, cellulose, microcrystal cellulose, microcrystalline cellulose, polyvinylpyrrolidone, water, methylhydroxybenzoate, propylhydroxybenzoate, talc, magnesium stearate, and mineral oil.

The composition according to the present invention may be prepared in an oral formulation, an external applicable formulation, a suppository formulation, or a sterilized injection solution formulation, such as powder, granule, tablet, suspension, emulsion, syrup, or aerosol, by using conventional methods.

The preparation may be performed using a diluent or excipient, such as a filler, an extender, a binder, a wetting agent, a disintegrant, or a surfactant. Examples of a solid formulation for oral administration are tablet, pill, powder, granule, capsule, etc. and these solid formulations are prepared by mixing with, for example, at least one excipient selected from the group consisting of starch, calcium carbonate, sucrose, lactose, and gelatin.

Also, in addition to such excipients, a lubricant, such as magnesium stearate or talc may also be used. As a liquid formulation for oral administration, suspension, oral liquid, emulsion, syrup, etc. may be used. Other than frequently used diluents, such as water or liquid paraffin, various other excipients, for example, a wetting agent, a sweetening agent, an aromatic agent, a preservation agent may be included.

Examples of a formulation for parenteral administration are a sterile aqueous solution, a non-aqueous solution, a suspension, an emulsion, a lyophilized formulation, and a suppository formulation. Examples of non-aqueous solution and suspension are propylene glycol, polyethylene glycol, vegetable oil, such as olive oil, and injectable ester, such as ethylolate. As a base compound for the suppository formation, witepsol, macrogol, tween 61, cacao oil, laurin oil, glycerogeratin, etc. may be used.

The dosage of an active ingredient in the composition according to the present invention may vary according to age, gender, and weight of a patient. For example, 0.1 to 100 mg/ kg of dosage may be given all at once or may be divided into several doses per day.

Also, the dosage of an active ingredient in the composition according to the present invention may be increased or reduced according to an administration path, a morbid state, gender, weight, age, etc. Accordingly, the dosage described above may not limit the scope of the present invention in any aspect.

The composition according to the present invention may be administered to a mammal, such as rat, mouse, livestock, or human being, via various administration paths. All of the administration methods are expectable, and examples thereof are oral administration, intrarectal or intravenous infusion, intramuscular infusion, subcutaneous infusion, and endocervical or intracerebroventricular injection.

In particular, if the composition according to the present invention is administered to a human body, adverse effects may not occur compared to other synthetic drugs because the composition is a natural extract, and stability thereof is guaranteed.

Also, the present invention also provides a health food for improving obesity or obesity related disease, wherein the health food includes as an active ingredient, an inhibitor that suppresses synthesis of a fatty acid that is over-expressed in a gender-specific serum. The fatty acid may include one or more selected from the group consisting of arachidic acid (C20:0) and eicosadienoic acid (C20:2n); derivatives of these fatty acids, and salts of these fatty acids.

The health food may be provided in powder form, granule form, tablet form, capsule form, syrup form, or drink form. The health food may include, other than the active ingredient, other foods or other food additives, and may be appropriately used according to a conventional method. An amount of the active ingredient included in the health food may depend on its purpose, for example, prevention, health maintenance, or therapeutic treatment.

An effective amount of the active ingredient included in the health food may be within an effective amount range of the composition. However, in the case of a long-term intake for the purpose of health maintenance and sanitation, or health control, the effective amount may be lower than the lower limit of the range. Because the active ingredient may not cause any problems against stability, an amount that is greater than the upper limit of the range may also be available.

The kind of the health food is not particularly limited, and examples thereof are meat, sausage, bread, chocolate, candy, snack, cracker, instant noodle, other noodles, chewing gums, dairy products including ice cream, various kinds of soups, beverages, tea, drinks, alcoholic beverages, and vitamin composite, etc.

The present invention also provides a method of screening an agent for treating obesity or obesity related disease, wherein the method includes treating a biological sample obtained from an individual that is needed to prevent or treat the obesity or obesity-related disease with a compound; and detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

The present invention also provides a method of diagnosing obesity or obesity-related disease, wherein the method includes detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids in a biological sample obtained from an individual that is needed to prevent or treat the obesity or obesity-related disease; and determining whether obesity or obesity related disease has been developed by comparing the expression profile with an expression profile with that of normal control group.

The present invention also provides a biomarker for evaluating a meat quality of livestock, wherein the biomarker includes a fatty acid that is specifically expressed in the gender-specific serum. The fatty acid may include one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

The biomarker may promote expression of FAT/CD36 gene, and may be specifically expressed in a gender-specific serum selected from female serum and castration serum.

The present invention also provides a method of evaluating meat quality of livestock, wherein the method includes detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids in a serum of livestock.

The present invention also provides a feed composition for fattening livestock, wherein the feed composition includes a fatty acid that is specifically over-expressed in the gender-specific serum as an active ingredient. The fatty acid may include one or more selected from the group consisting of fatty acids selected from arachidic acid (C20:0) and eicosadienoic acid (C20:2n); derivatives of these fatty acids, and salts of these fatty acids. The fatty acids and derivatives or salts thereof may contribute to marbling production by promoting induction of differentiation from a muscle stem cell to adipocyte.

The feed composition for fattening livestock to improve a meat quality may be included in an amount of 0.01 to 1 wt% with respect to a concentrated feed or a formulated feed. If the amount of the feed composition is less than 0.01 wt%, the addition effect may be negligible, and if amount of the feed composition is greater than 1 wt%, the addition costs are too high and thus the economical effect in view of the costs is low.

Also, the feed composition for fattening livestock to improve a meat quality may be fed to a ruminant in an amount of 1 to 120 g/per day/per head (an amount supplied to a ruminant per day). The feed amount may also depend on various conditions, such as weight of a ruminant or a feed condition.

### [Mode for Invention]

The present invention will be described in further detail with reference to the following examples. These examples are for illustrative purposes only and are not intended to limit the scope of the present invention.

### <Example 1> Isolation of gender-specific serum

### 1. Experiment materials

A -20°C freezer, a high-speed cold centrifuger (GRX-220; TOMY TECH, California, USA), 0.2µm and 0.8µm disposable syringe filters (Advantec, Seoul, Korea), a 10Mℓ disposable syringe (Whagi Medical, Seoul, Korea), and filter paper (#2, #5; Advantec, Seoul, Korea) were used.

### 2. Extraction method

Korean bovine or porcine sample was collected immediately after slaughtered of animals from slaughter house by using a sterilized vessel in which an anticoagulant was not added. The collected blood sample was delivered to a laboratory within one hour and then centrifuged at 5,000 rpm for 20 minutes and tranquilized at a temperature of 4°C for 25 to 30 hours and the supernatant was collected and quickly frozen at a temperature of -20°C.

The quickly frozen sample was thawed at room temperature and centrifuged at 7,000 rpm for 30 minutes and the supernatant was inactivated at a temperature of 56°C for 30 minutes. Then, the serum was filtered through #2 and 5 filter paper, followed by filtering with a 0.8µm filter and a 0.2µm filter in the stated order, thereby extracting and refining the serum.

### <Example 2> Analysis on steroid content in extracted serum

Amounts of the respective steroids in the extracted serum were analyzed using an Estradiol, estrone, and testosterone ELISA kits. i.e, a control group, the serum sample prepared according to Example 1, and a reference material was divided and added into each well and then an enzyme conjugate was added thereto. Then, each well was tranquilized at room temperature under cold conditions.

Then, the analysis plate was rinsed three times with a wash buffer and a substrate solution was added thereto, and the resultant plate was tranquilized for 30 minutes. After 15 minutes, a fixing solution was added thereto and absorption thereof was measured at a wavelength of 450 nm. Results thereof are shown in Table 1 below.

Regarding estradiol and estrone, female bovine serum (FS) > male bovine serum (MS), castrated male bovine serum (C-MS) > FBS, and regarding testosterone, MS > C-MS, FS> FSB. The extracted serum had more steroid hormone than the FBS, and an amount of the steroid hormone in serum varied according to an individual.

**[Table 1]**

| | Estradiol (pg/mL) | Estrone (pg/mL) | Testosterone (ng/mL) |
|---|---|---|---|
| FBS | 19.2 | 202.1 | 0.35 |
| FS | 36.9-273.3 | 147.6-9598.2 | 0.8-10.72 |
| MS | 152.8 | 2426.8 | 23.69 |
| C-MS | 150.2 | 2567.8 | 4.71 |

### <Example 3> Primary culture of Korean bovine myogenic satellite cell by using extracted serum

A FBS and the serum prepared according to Example 1, i.e, the Korean bovine female, male, and castrated male sera were used. In detail, for each, 10% serum-supplemented dulbecco's modified eagle's medium (DMEM) was added to a myogenic satellite cell in a 100x20mm dish (Falcon, USA), and incubation was performed thereon in a incubator in an atmosphere of 5% CO₂ and at a temperature of 37°C. 6 hours after the incubation, cells that were not attached to the bottom were removed and then, the culture media was changed with a fresh DMEM culture after every 48 hours. The incubation was performed for 7 days.

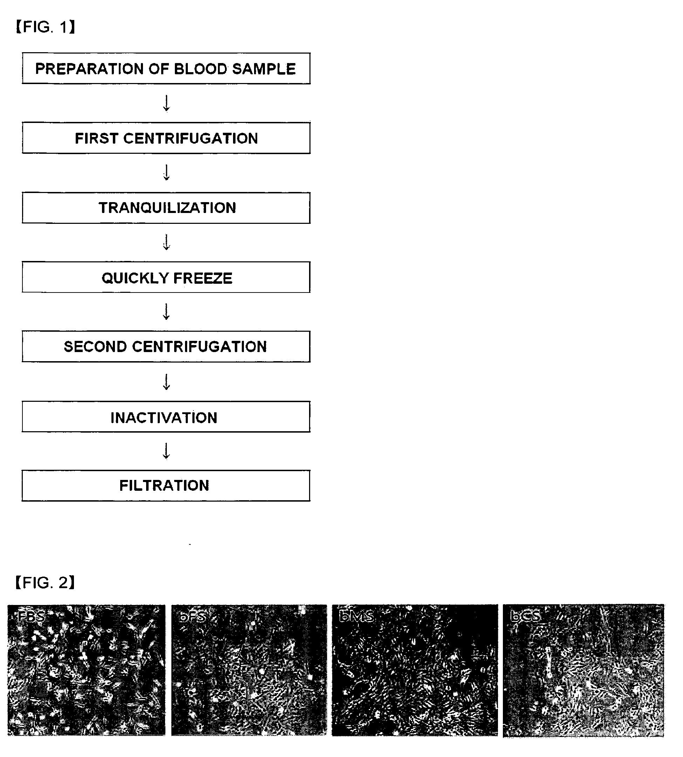

As a result, as illustrated in FIG. 2, regarding the primary culture of myogenic satellite cells, cell proliferation in the respective sera showed the following results: male(bMS) > castrated male (bCS) > female (bFS) > FBS.

### <Example 4> cell proliferation using extracted serum

To confirm the effect of the previously prepared female, male, and castrated male sera including a FBS on cell proliferation in various kinds of cells, 10% each serum was added to DMEM, followed by incubation in an incubator in an atmosphere of 5% CO₂ and at a temperature of 37°C. Three days of the incubation, cells were collected and enumerated using a hemotocytometer. The enumerating was performed at least three times.

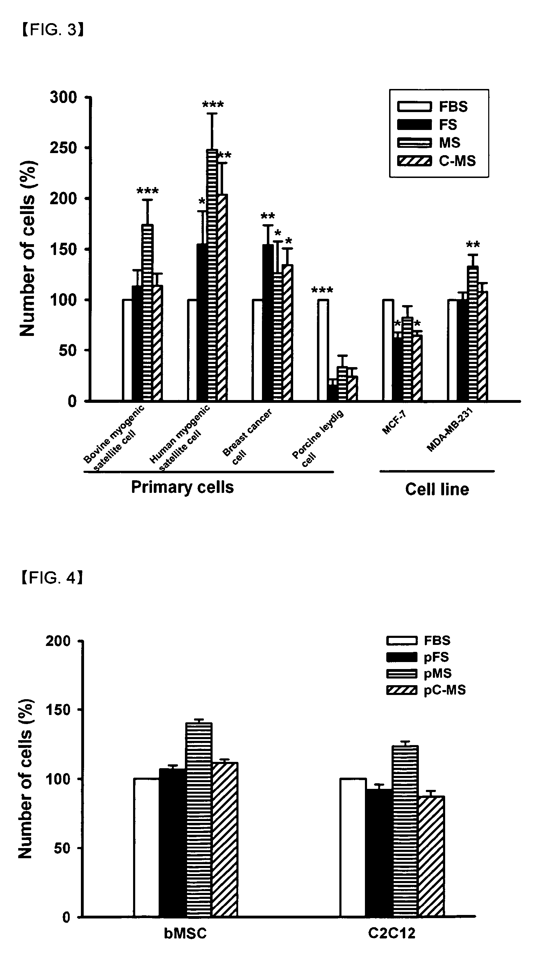

As a result, as illustrated in FIGS. 3 and 4, regarding Korean bovine and porcine cases, the number of cells was higher than in the case with FBS. In particular, when cultured in the male serum, the number of myogenic satellite cells was relatively high.

### <Example 5> Observation on relationship between lipid accumulation and hormone according to muscle differentiation

To confirm a relationship between a lipid accumulation level and hormone in myotube formed cells, cells were grown in DMEM (phenol-red (-))/10% FBS and then, each of the cells was treated with 17β-estradiol, testosterone, and [17β-estradiol + testosterone] for about 2 weeks. Then, lipid accumulation levels of the cells were confirmed.

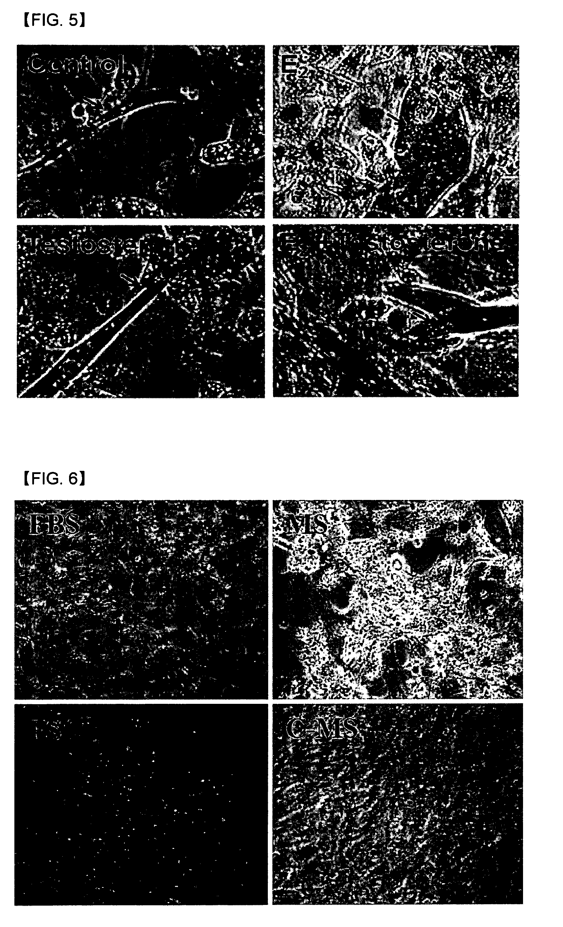

As a result, as illustrated in FIG. 5, the lipid accumulation level was highest in myotube of the cells treated with 17β-estradiol, and decreases in the following order of [17β-estradiol + testosterone], testosterone, and the control group that was not treated with hormone.

### <Example 6> Trans-differentiation from myogenic satellite cell to adipocyte

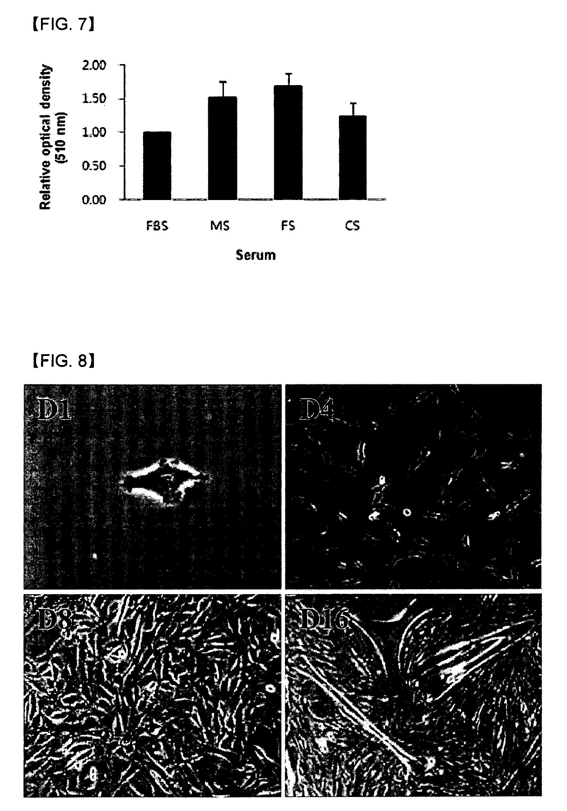

The lipid accumulation level in cells was confirmed by inducing trans-differentiation in bovine myogenic satellite cells by using FBS and the previously prepared female, male, and castrated male sera. As a result, as illustrated in FIGS. 6 and 7, lipid accumulation in the respective cells showed the following results: female (FS) > male(MS) > castrated male (CS) > FBS. This result is consistent with the result shown in FIG. 5, and it was confirmed that the cell culture time was reduced to about 1 week and the lipid accumulation level was very high.

Example 6 is presented herein to describe how difficult the steroid hormone experiment is to be carried out, and Example 6 shows that in Example 5, the extracted and purified serum according to the present invention can be used instead.

### <Example 7> Transdifferentiation of MSC into ALC

### 1) MSC isolation and primary culture

Male bovine hind limb skeletal muscles (24-26 weeks, 550-600 kg of body weight) were collected from a regional slaughter house, washed with phosphate buffered saline (PBS), and minced into fine pieces using sterilized scissors. The minced tissues were digested by trypsin-EDTA(GIBCO, Carlsbad, CA, USA) for 2 hours, and centrifuged at 90 g for 3 minutes. The upper portion was filtered using a vessel having a 40µm diameter pore size and the filtrate was centrifuged at room temperature at 2,500 rpm for 20 minutes. The digestion medium was removed, leaving the cell precipitate at the bottom of the tube. The collected cell precipitate was washed three times using DMEM (HyClone Laboratories, Logan, UT, USA) containing 1% penicillin/streptomycin (Invitrogen, Carlsbad, CA, USA), and cultured in a 100 mm-diameter culture dish using DMEM supplemented with 10% fetal bovine serum (FBS; HyClone Laboratories), 1% penicillin/streptomycin, and 0.1% amphotericine (GIBCO) by incubating at 37°C in an atmosphere of 5% CO₂.. The condition of primary MSC culture was checked daily and the medium was changed every second day.

### 2) Single cell culture

Bovine MSC culture was trypsinized and the cells were collected and washed for three times. Then, single cells were collected using a micro-pipette and each was transferred into a well of a 12-well plate with a micro-drop of culture medium under microscope examination. Each cell was cultured with a micro-drop of medium until the cell attached to the culture dish and 1 ml of medium was added. Cell morphology and proliferation was observed everyday using a light microscope (Nikon, Tokyo, Japan).

### 3) Results

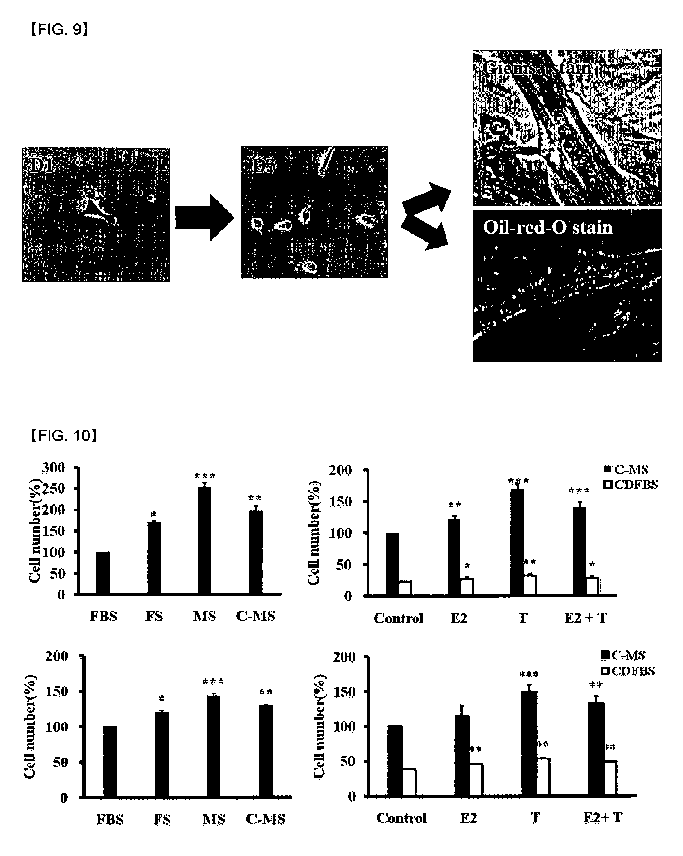

MSC was separated from the bovine hind limb skeletal muscles. Purity of the separated MSC was confirmed by single cell culture, and the introduced single cells form a multi-cell and fuse each other to form myotubes. Overall, 96 single cell culture were formed, and among them, 80 (83.3%) formed myotubes (FIG. 8). When a single MSC individually progressed into a multi-cell is subcultured, in the case of a general medium, the subcultured cell is differentiated into a myotube, and in the case of a lipid formation medium, the subcultured cell is transdifferentiated into ALC. The cell cultured in the general medium, once stained by Giemsa stain, syncytium was observed. Several nuclei were observed in the fused cell, and this result shows that myotubes were formed in the culture starting from a single cell. When the medium is replaced with liquid formation medium after the formation of muscle, muscle droplets were formed in myotubes, and the formation was confirmed by positive ORO staining (FIG. 9).

### <Example 8> MSC proliferation effect according to gender-specific serum

### 1) Subculturing

To confirm the effect of the gender-specific serum prepared according to Example 1, primary MSC culture was subcultured to passage 1 when the primary culture reached confluence. The cultured cells were harvested by trypsin-EDTA, and subcultured in wells of 6-well plates with DMEM supplemented with FBS after washing the collected cell precipitate. Cells were cultured for a day in the supplemented medium to allow for equal attachment in the culture dish. The next day the medium was removed and replaced with DMEM supplemented with 10% FBS, MS, FS and C-MS. The effect of Cell proliferation, differentiation to myotubes, and transdifferentiation to ALCs according to a gender-specific serum was confirmed.

### 2) Hormone treatment

MSCs were cultured in DMEM supplemented with C-MS to observe the steroidal effect on the cultured muscle cells. E₂, testosterone, or a combination of E₂ and testosterone (all 10 nM) were added directly to each medium. Cells were enumerated under microscopic examination using a hematocytometer.

### 3) MSC differentiation effect

For MSC differentiation, cells were collected from a primary culture dish and subcultured. The cells in passage one were cultured in medium supplemented with FBS, MS, FS, or M-CS for three days, and cell numbers in each culture were enumerated. The number of cells was the highest in the medium supplemented with MS, followed by supplementation with C-MS, FS, and FBS (see the upper left side of FIG. 2).

MSC were subcultured in C-MS or CDFBS and treated with 10 nM of E₂ or testosterone, or a combination of E₂ and testosterone. Treatment with the steroids increased cell proliferation compared to the respective control, with the increment of cell proliferation being highest in the testosterone-treated culture followed by the combination E₂ and testosterone, and E₂ only (see the lower right side of FIG. 2).

In addition, the effect of different serum and steroids on the C2Cl2 myoblast cell line was also observed. The data generated were in agreement with those of bovine MSCs. MS and testosterone-treated C-MS and CDFBS markedly increased C2C12 cell proliferation (see the lower left and right sides of FIG. 2).

### <Example 9> Myogenesis-related gene expression

### 1) Immunocytochemical analysis

Isolated MSCs were cultured in a covered glass-bottom dish and allowed to differentiate into myotubes or to transdifferentiate into ALCs under different serum conditions. Then, the culture mediun was removed and cells were rinsed with PBS. PBS rinsing was also performed between all subsequent steps. Cells were fixed with 4% formaldehyde, permeabilized by 0.2% Triton X-100 and 3-4 drops of Image-iT^{™} FX signal enhancer (Invitrogen) was applied. Primary antibody (mouse monoclonal IgG₁ Myogenin; sc-12732, rabbit polyclonal IgG CD36 sc-9154; Santa Cruz Biotechnology, Santa Cruz CA, USA) was added and incubated at 4 °C in a humid environment overnight, and then incubated with secondary antibody (Alexa Fluor 488 goat anti-mouse SFX kit and Alexa Fluor 488 goat anti-rabbit SFX kit; Molecular Probes, Eugene, OR, USA) at room temperature for 1 hour. Each cell nucleus was counterstained by 4' 6'-diamino-2-phenylindole (DAPI; Sigma-Aldrich, St. Louis MO, USA). Immunostained cells were observed using a fluorescence microscope (Nikon).

### 2) Real-time RT-PCR analysis

Trizol^{™} reagent (Invitrogen) was used to extract total RNA from cells, according to the manufacturer's protocol. RNA was tranquilized in diethylpyrocarbonate-treated water at - 80 °C. Concentrations of the extracted RNA samples were measured using an ND-100 spectrophotometer (NanoDrop Technologies, Wilmington, DE, USA) and the purity was checked with an Agilent 2100 bioanalyzer (Agilent Technologies, Palo Alto, CA, USA) prior to RT-PCR. RNA was reverse-transcribed into the first stranded cDNA using Superscript-II reverse transcriptase (Invitrogen). Total RNA (1 µg in 20 µl total volume) was primed with oligo (dT) 20 primers (Bioneer, Daejeon, Korea), and reverse transcription was carried out at 42 °C for 50 min and 72 °C for 15 min. Subsequently, 2 µl of the 10× diluted cDNA product and 10 pmoles of each gene-specific primer were used to perform PCR using a 7500 real-time PCR system (Applied Biosystems, Foster City, CA, USA). Power SYBR® Green PCR Master Mix (Applied Biosystems) was used as the fluorescence source. Primers were designed with Primer 3 software (http://frodo.wi.mit.edu) using sequence information listed at the National Center for Biotechnology Information. The following primers shown in Table 2 below were used.

**[Table 2]**

| Protein | Forward primer | Reverse primer | |
|---|---|---|---|
| GAPDH | 5'-gggtcatcatctctgcacct-3' | 5'-acagtcttctgggtggcagt-3' | |
| ER-a | 5'-caggtgccctattacctgga-3' | 5'-gcctgaggcatagtcattgc-3' | |
| AR | 5'-tctcccaagaatttggatgg-3' | 5'-ggagcttggtgagctggtag-3' | |
| Desmin | 5'-tgtcgaaaccagacctcaca-3' | 5'-gtggcggtactccatcatct-3' | 57 °C |
| Myogenin | 5'- tgggcgtgtaaggtgtgtaa-3' | 5'-tgcaggcgctctatgtactg-3' | 57 °C |
| FAT/CD36 | 5'-agatgcagcctcatttccac-3' | 5'-gcaaaagcaaaggatggaag-3' | |

The real-time PCR was carried out under the following conditions: pre-denaturation of the synthesized cDNA at 95°C for 10 min was followed by 40 cycles of denaturation at 95°C for 33 s, annealing at each gene-specific primer Tm (°C), and extension at 72°C for 33 s. Proper amplification of the genes of interest was verified by melting point analysis and 1.2% agarose gel electrophoresis.

### 3) Results

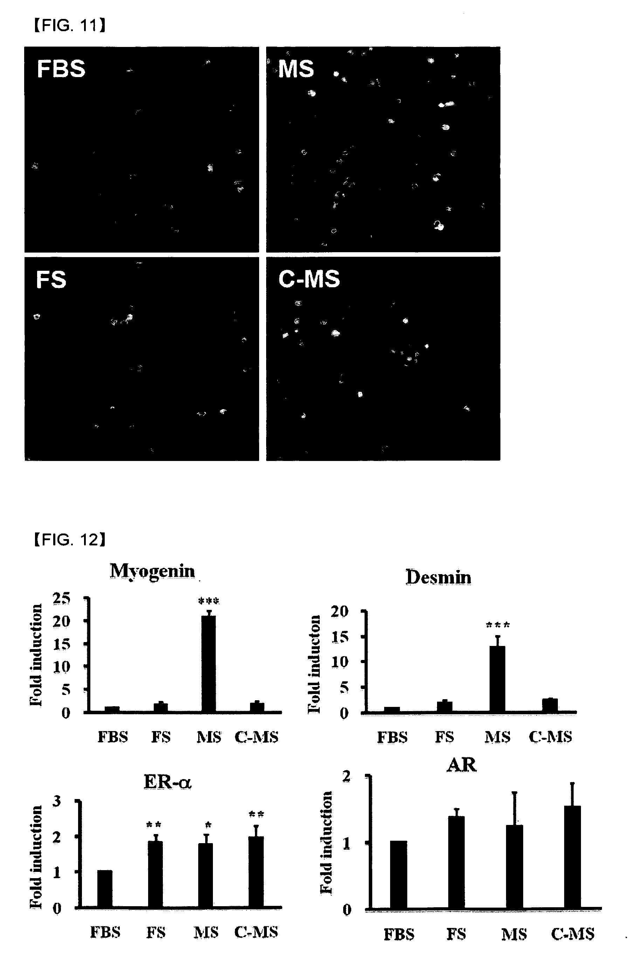

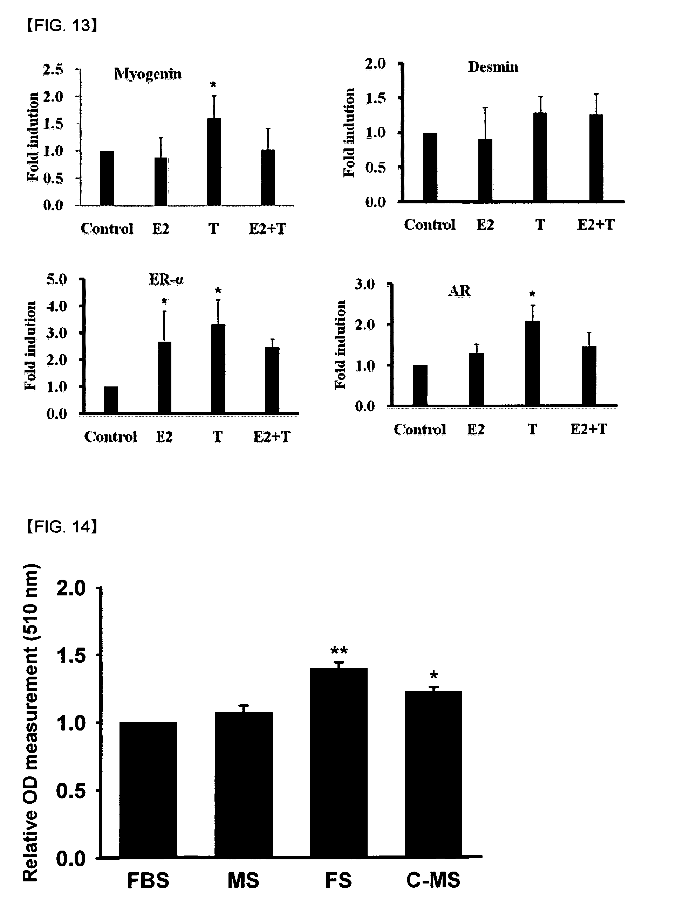

According to immunocytochemical analysis of myogenesis in myotube forming cells cultured in media supplemented with different gender-specific sera, a marker gene for myogenesis showed the strongest suppression on the myogenesis expression in cells cultured in medium supplemented with MS, followed by FS, C-MS, and FBS (FIG. 11). mRNA expression of desmin and myogenin was analyzed by real time RT-PCR in cells cultured in media supplemented with the different sera. The highest expression of both myogenin and desmin was evident in cells cultured in the presence of MS. In addition, expression of ER-α and AR were also analyzed. Expression of ER-α was significantly increased in FS-, MS- and C-MS-supplemented cultures. However, AR expression was not significantly different among the cells cultured in the different sera (FIG. 12). Testosterone up-regulated myogenin and AR expression significantly and there was significant up-regulation of ER-α by the combined E₂ and testosterone treatment. No significant change in expression of desmin was observed after hormone treatment (FIG. 13).

### <Example 10> MSC transdifferentiation evaluation

### 1) Oil-red-O staining analysis

A stock solution of Oil-Red-O (ORO; Sigma-Aldrich) was prepared by mixing 5 mg/ml of ORO in 100% isopropanol (Merck, Darmstadt, Germany). Working solution was made by diluting the stock solution to 6:4 stock solutions to autoclaved de-ionized water. The working solution was filtered through Whatman filter paper (Whatman International, Maidstone, UK) before use. The formalin fixed cells were washed three times using autoclaved de-ionized water. The working solutions of ORO (1 ml/well) were added and, 15 minutes later, the staining solution was removed and each well was washed using autoclaved de-ionized water. After air-drying, deionized water was added to each well and the cells were observed under a light microscope equipped with a digital camera. After acquiring photographs, deionized water was removed and the cells were destained with 100% isopropanol for 10 min. Optical density was measured at 510 nm using a VersaMax microplate reader (Sunnyvale, CA, USA).

### 2) Results

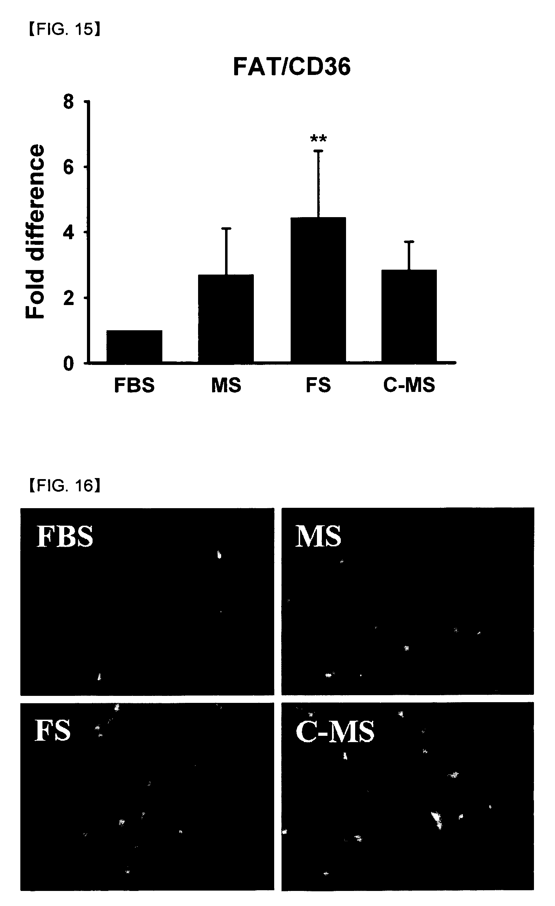

MSCs were transdifferentiated into ALCs by switching the culture medium into adipogenic medium. To observe the effect of different sera on transdifferentiation, cells were cultured in adipogenic media supplemented with different sera for 7 days and stained with ORO. Photographic examination (FIG. 6) and spectrophotometric quantification of ORO staining (FIG. 7) indicated the highest transdifferentiation of MSCs was in the culture supplemented with FS followed by C-MS, MS, and FBS. Similarly, the gene related to the fatty acid transporter FAT/CD36 was significantly upregulated in the FS-supplemented adipogenic medium compared to the other serum-supplemented adipogenic media (FIG. 15). In contrast, expression of genes related to lipogenesis, adipose differentiation binding protein, CCAAT/enhancer binding protein, and fatty acid binding protein expression were similar in cells cultured in the different sera. FAT/CD36 protein expression was observed by immunocytochemistry, and was also highest in the FS-supplemented medium (FIG. 16).

### <Example 11> Analysis on fat that is accumulated the most under the influence of E₂

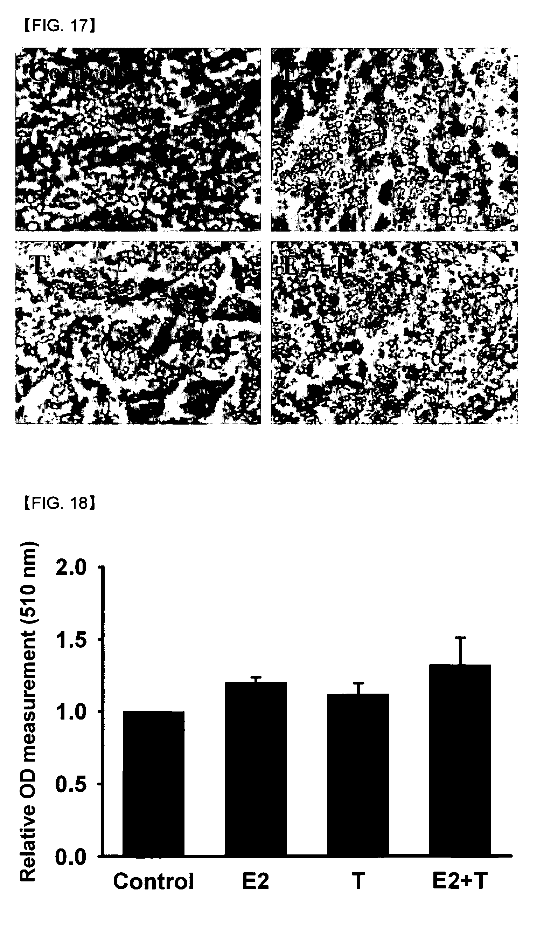

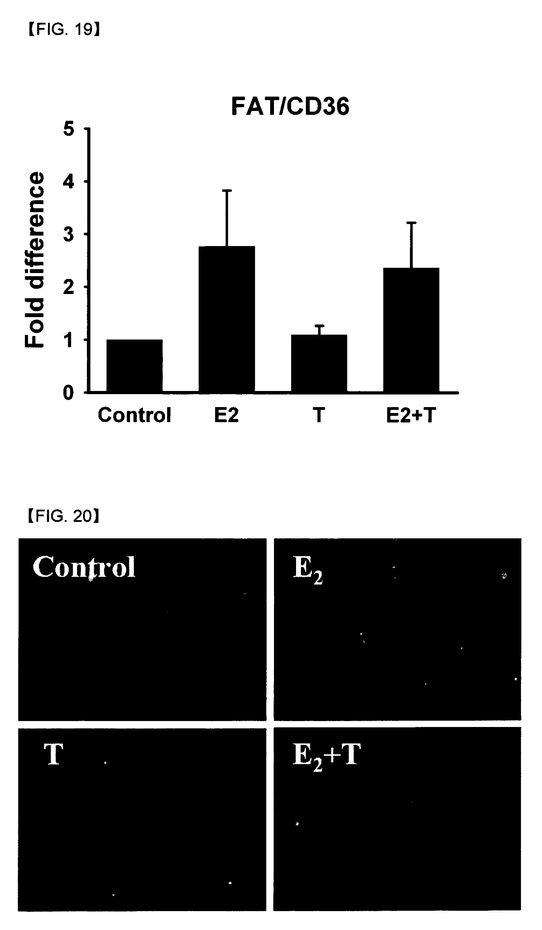

To confirm that the highest lipid accumulation was due to the female steroid hormone in FS, isolated MSCs were cultured in adipogenic medium supplemented with C-MS to check the steroidal effect in the muscle cells. The cells were cultured in adiopogenic medium treated with E₂, testosterone, or E₂ and testosterone. The cells were stained with ORO to check the accumulation of lipid droplets. Photographic examination (FIG. 17) and spectrophotometric quantification of ORO stains (FIG. 18) showed the slight increase of lipid droplets in hormone-treated cells compared to untreated. mRNA expression showed significant increase of FAT/CD36 expression in E₂-treated culture (FIG. 19). FAT/CD36 protein expression was upregulated by E₂ treatment (FIG. 20).

### <Example 12> Fatty acid contents analysis in gender-specific sera

### 1) Fatty acid analysis

For fatty acid analysis, total lipids were extracted from 0.5 ml of serum with 5 ml of a hexane: isopropanol mixture and 2 ml of 6.7% Na₂SO₄ solution. Two hundred and fifty µl of pentadecanoic acid (C15:0) was spiked as an internal standard. Extracted total lipids were methylated with 12.5% boron trifluoride in methanol according to modified Morrison and Smith method (18). Methylated lipids were dissolved with 500 µl of hexane and 1 µℓof lipid samples were injected to Agilent 7890 gas chromatography system (Agilent technology Inc., Santa Clara, CA, USA) equipped with HP-5 column. Helium was used as a carrier gas with 1 ml/min of flow rate. Separated fatty acids were detected and quantified using Agilent 5975 GC/MSD detector and MSC chemstation software (Agilent technology Inc.). For standard, Supelco™ 37 Component FAME mix (Supelco, Bellefonte, PA, USA) was used.

### 2) Results

Fatty acid contents among different sera were analyzed (Table 3 below). Among injected thirty seven fatty acids, only thirty fatty acids were exactly matched with MSD chemstation data base. The concentration of undecanoic acid (C11) in C-MS was higher than that in FS with significance, but undecanoic acid was not detected from the male sera. There were no significant differences (P > 0.05) in the concentrations of saturated fatty acids containing less than 18 carbons among gender. The concentrations of tricosanoic acid (C24:0) in MS and C-MS were higher than that in FS with significance (P < 0.05). Whereas, the concentrations of arachidic acid (C20:0) and eicosadienoic acid (C20:2n) in FS were significantly higher than those in MS and C-MS.

**[Table 3]**

| Fatty acid | MS (mg/mL) | FS (mg/mL) | C-MS (mg/mL) |
|---|---|---|---|
| Caprylic acid (C8) | 0.64± 0.13 | 0.46± 0.14 | 0.88± 0.14 |
| Carpric acid (C10) | 1.00± 0.19 | 0.59± 0.13 | 0.80± 0.11 |
| Undecanoic acid (C11) | nd | 0.22± 0.15^{a} | 1.13± 0.15 |
| Lauric acid (C12) | 3.32± 0.17 | 1.56± 0.24 | 3.78± 0.33 |
| Tridecanoic acid (C13) | 0.32± 0.15 | nd | 1.39± 0.27 |
| Myristic acid (C14:0) | 16.86±0.33 | 15.05± 0.53 | 17.92± 0.52 |
| Methyl E-11 tetradecenoate (C14:1) | -2.16± 0.21 | 1.41± 0.20^{a} | 2.76± 0.27 |
| Palmitic acid (C16) | 369.14± 1.41 | 355.51± 2.47 | 364.30± 2.52 |
| 9- Hexadecenoic acid (C16:1) | 45.38± 1.17 | 47.82± 0.92 | 46.01± 1.34 |
| Heptadecanoic acid (C17) | nd | nd | nd |
| Stearic acid (C18:0) | 1137.95± 2.42 | 982.10± 3.22 | 964.49± 4.17 |
| 9- octadecenoic acid (C18:1-cis) | 147.47± 1.24 | 214.25± 2.51 | 225.5± 2.61 |
| 9- octadecenoic acid (C18:1-trans) | 198.77± 2.32 | 177.37± 1.83 | 192.90± 1.97 |
| Linolenic acid (C18:2n-cis) | nd | nd | nd |
| 9,12- octadecadienoic1854.53± acid (C18:2n-trans) | 6.06 | 1314.35± 5.96 | 1381.56± 6.54 |
| α- linolenic acid (C18:3n) | nd | nd | nd |
| Arachidic acid (C20:0) | 3.25± 0.32 | 7.17± 0.37^{a} | 2.38t± 0.23 |
| Eicosenoic acid (20:1n) | 5.53± 0.25 | 7.18± 0.20 | 5.36± 0.22 |
| Eicosadienoic acid (C20:2n) | 12.81± 0.39 | 22.48± 0.63^{a} | 10.43± 0.40 |
| Eicosatrienoic acid (C20:3n3) | 242.55± 2.60 | 351.66± 2.90 | 367.90± 3.90 |
| Arachidonic acid (C20:4n6) | 234.21± 0.73 | 234.56± 1.47 | 279.90± 2.02 |
| Eicosapentaenoic acid (C20:5n3) | 157.02± 3.18 | 387.57± 3.40 | 177.37± 3.63 |
| Heneicosanoic acid (C21:0) | nd | nd | nd |
| Erucic acid (C22:1n) | 93.13± 1.40 | 124.71± 1.26 | 180.22± 1.91 |
| Behenic acid (C22:2) | 36.00± 1.25^{a} | 47.90± 0.83^{a} | 75.14± 1.09 |
| Docosahexaenoic acid (C22:6n3) | 29.32± 0.51 | 53.07± 0.77 | 53.56± 1.07 |
| Tricosanoic acid (C23:0) | 81.84± 1.67^{a} | 80.93± 0.93^{a} | 142.86± 1.53 |
| Tetracosanoic acid (C24 :0) | 43.45± 1.14 | 33.41± 0.93^{a} | 66.62± 1.07 |
| Nervonic acid (C24:1) | 85.78± 1.67^{a} | 90.72±1.02^{a} | 154.75±1.59 |

### [Sequence List Text]

SEQID NO: 1 and SEQID NO: 2 constitute a forward and reverse primer set for amplifying GAPDH,
SEQID NO: 3 and SEQID NO: 4 constitute a forward and reverse primer set for amplifying ER-a,
SEQID NO: 5 and SEQID NO: 6 constitute a forward and reverse primer set for amplifying AR,
SEQID NO: 7 and SEQID NO: 8 constitute a forward and reverse primer set for amplifying desmin,
SEQID NO: 9 and SEQID NO: 10 constitute a forward and reverse primer set for amplifying myogenin, and
SEQID NO: 11 and SEQID NO: 12 constitute a forward and reverse primer set for amplifying FAT/CD36.

## Claims

1. A method of preparing a gender-specific serum, the method comprising:
preparing blood from a mammal that is discarded after slaughter;
first centrifuging the prepared blood;
tranquilizing after the centrifugation;
quickly freezing a collected supernatant;
thawing the resultant product at room temperature, followed by second centrifuging;
inactivating the supernatant: and
filtering the inactivated supernatant.

2. The method of claim 1, wherein the gender-specific serum is any one selected from the group consisting of a female serum, a male serum, and a castrated male serum.

3. The method of claim 1, wherein the first centrifuging is performed at 4,000 to 6,000 rpm for 10 to 30 minutes to increase cohesiveness.

4. The method of claim 1, wherein the tranquilizing is performed at a temperature of 0 to 10°C for 25 to 30 hours.

5. The method of claim 1, wherein the second centrifugation is performed at 6,000 to 8,000 rpm for 20 to 40 minutes to allow the filtering to be easily performed.

6. The method of claim 1, wherein the inactivating is performed at a temperature of 45 to 65°C for 20 to 40 minutes.

7. The method of claim 1, wherein the filtering is performed using a filter paper, a syringe filter, or a combination thereof.

8. A biomarker for diagnosing obesity or obesity related disease, the biomarker comprising a fatty acid that is specifically expressed in the gender-specific serum of any one of claims 1-7.

9. The biomarker of claim 8, wherein the fatty acid comprises one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

10. The biomarker of claim 8 or claim 9, wherein the obesity-related disease is selected from the group consisting of diabetes, insulin resistant syndrome, hyperlipemia, ostarthritis, lipodystrophy, nonalcoholic steatohepatitis, cardiovascular disease, polycystic ovary syndrome, and metabolic syndrome.

11. The biomarker of claim 8 or claim 9, wherein the biomarker promotes expression of a FAT/CD36 gene.

12. A composition for treating or preventing obesity or obesity related disease, the composition comprising an inhibitor that prevents synthesis of a fatty acid that is specifically over-expressed in the gender-specific serum of any one of claims 1-7.

13. The composition of claim 12, wherein the fatty acid comprises one or more selected from the group consisting of a fatty acid selected from arachidic acid (C20:0) and eicosadienoic acid (C20:2n); derivatives of these fatty acids, and salts of these fatty acids.

14. The composition of claim 13, wherein the inhibitor comprises a FAT/CD36 inhibitor.

15. The composition of claim 14, wherein the FAT/CD36 inhibitor is selected from the group consisting of siRNA that inhibits expression of a FAT/CD36 gene and an antibody that specifically bonds to the FAT/CD36 gene.

16. The composition of claim 14 or claim 15, wherein the FAT/CD36 inhibitor suppresses induction of differentiation from muscle stem cells into adipocyte.

17. A method of screening an agent for treating obesity or obesity related disease, the method comprising:
treating a biological sample obtained from an individual that is needed to prevent or treat the obesity or obesity-related disease with a compound; and
detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

18. A method of diagnosing obesity or obesity-related disease, the method comprising:
detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0),
tetracosanoic acid (C24:0), and nervonic acid; derivatives of these fatty acids, and salts of these fatty acids in a biological sample obtained from an individual that is needed to prevent or treat the obesity or obesity-related disease; and
determining whether obesity or obesity related disease has been developed by comparing the expression profile with an expression profile of that of a normal control group.

19. A biomarker for evaluating a meat quality of livestock, the biomarker comprising a fatty acid that is specifically expressed in the gender-specific serum of any one of claims 1-7.

20. The biomarker of claim 19, wherein the fatty acid comprises one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24:0), and nervonic acid; derivatives of these fatty acids, and salts of these fatty acids.

21. The biomarker of claim 19 or claim 20, wherein the biomarker is used to expect or diagnose marbling production in livestock.

22. A method of evaluating a meat quality of livestock, the method comprising detecting an expression profile of one or more selected from the group consisting of fatty acids selected from undecanoic acid (C11), methyl E-11-tetradecenoate (C14:1), arachidic acid (C20:0), eicosadienoic acid (C20:2n), behenic acid (C22:2), tricosanoic acid (C23:0), tetracosanoic acid (C24 :0), and nervonic acid; derivatives of these fatty acids, and salts of these fatty acids in a serum of livestock.

23. A feed composition for fattening livestock, the feed composition comprising a fatty acid that is specifically over-expressed in the gender-specific serum of any one of claims 1-7 as an active ingredient.

24. The feed composition of claim 23, wherein the fatty acid comprises as the active ingredient one or more selected from the group consisting of fatty acids selected from arachidic acid (C20:0) and eicosadienoic acid (C20:2n); derivatives of these fatty acids, and salts of these fatty acids.


SEQUENCE LISTING
<110>    Industry-Academic Cooperation Foundation, Yeungnam University

<120>    Preparation method of gender-specific serum and biomarker using
         the same

<130>    OP-2010-0010PCT

<160>    12

<170>    KopatentIn 1.71

<210>    1
<211>    20
<212>    DNA
<213>    Artificial Sequence

<220>
<223>    Forward primer for glyceraldehyde 3-phosphate dehydrogenase
         (GAPDH)

<400>    1
gggtcatcat ctctgcacct                                                   20

<210>    2
<211>    20
<212>    DNA
<213>    Artificial Sequence

<220>
<223>    Reverse primer for glyceraldehyde 3-phosphate dehydrogenase
         (GAPDH)

<400>    2
acagtcttct gggtggcagt                                                   20

<210>    3
<211>    20
<212>    DNA
<213>    Artificial Sequence

<220>
<223>    Forward primer for ER-alpha

<400>    3
caggtgccct attacctgga                                                   20

<210>    4
<211>    20
<212>    DNA
<213>    Artificial Sequence

<220>
<223>    Reverse primer for ER-alpha

<400>    4
gcctgaggca tagtcattgc                                                   20

<210>    5
<211>    20
<212>    DNA

<213>    Artificial Sequence

<220>
<223>    Forward primer for AR


<400>    5
tctcccaaga atttggatgg                                                    20


<210>    6
<211>    20
<212>    DNA
<213>    Artificial Sequence

<220>
<223>    Reverse primer for AR


<400>    6
ggagcttggt gagctggtag                                                    20


<210>    7
<211>    20
<212>    DNA
<213>    Artificial Sequence

<220>
<223>    Forward primer for desmin


<400>    7
tgtcgaaacc agacctcaca                                                    20


<210>    8
<211>    20
<212>    DNA
<213>    Artificial Sequence

<220>
<223>    Reverse primer for desmin


<400>    8
gtggcggtac tccatcatct                                                    20


<210>    9
<211>    20
<212>    DNA
<213>    Artificial Sequence

<220>
<223>    Forward primer for myogenin


<400>    9
tgggcgtgta aggtgtgtaa                                                    20


<210>    10
<211>    20
<212>    DNA
<213>    Artificial Sequence

<220>

<223>    Reverse primer for myogenin

<400>    10
tgcaggcgct ctatgtactg                                                    20

<210>    11
<211>    20
<212>    DNA
<213>    Artificial Sequence

<220>
<223>    Forward primer for FAT/CD36

<400>    11
agatgcagcc tcatttccac                                                    20

<210>    12
<211>    20
<212>    DNA
<213>    Artificial Sequence

<220>
<223>    Reverse primer for FAT/CD36

<400>    12
gcaaaagcaa aggatggaag                                                    20